(19) **Europäisches Patentamt**
**European Patent Office**
**Office européen des brevets**

(11) **EP 4 129 279 A1**

(12) **EUROPEAN PATENT APPLICATION**
published in accordance with Art. 153(4) EPC

(43) Date of publication:
**08.02.2023 Bulletin 2023/06**

(21) Application number: **21774136.2**

(22) Date of filing: **25.03.2021**

(51) International Patent Classification (IPC):
***A61K 31/194*** (2000.01)     ***A61K 9/20*** (1974.07)
***A61P 13/12*** (2000.01)

(52) Cooperative Patent Classification (CPC):
**A61K 9/20; A61K 31/194; A61P 13/12**

(86) International application number:
**PCT/JP2021/012668**

(87) International publication number:
**WO 2021/193856 (30.09.2021 Gazette 2021/39)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.03.2020  JP 2020056660**

(71) Applicants:
• **Tohoku University**
  **Sendai-shi, Miyagi 980-8577 (JP)**
• **Nippon Chemiphar Co., Ltd.**
  **Tokyo 101-0032 (JP)**

(72) Inventors:
• **ABE Michiaki**
  **Sendai-shi, Miyagi 980-8577 (JP)**
• **KOSHIBA Seizo**
  **Sendai-shi, Miyagi 980-8577 (JP)**
• **NISHIOKA Koichiro**
  **Tokyo 101-0032 (JP)**
• **YAMASAKI Satomi**
  **Tokyo 101-0032 (JP)**
• **SAKURAI Tetsuya**
  **Tokyo 101-0032 (JP)**
• **NAKAI Toshiki**
  **Tokyo 101-0032 (JP)**

(74) Representative: **V.O.**
  **P.O. Box 87930**
  **2508 DH Den Haag (NL)**

(54) **RENAL FUNCTION PROTECTANT**

(57)    To provide a medicine useful for protecting renal function or reducing the dietary acid load on the kidney in mammals, particularly humans.

Provided is a pharmaceutical composition containing citric acid, a pharmaceutically acceptable salt of citric acid, a hydrate of citric acid, a hydrate of the pharmaceutically acceptable salt of citric acid, or a mixture thereof. Administration of the present pharmaceutical composition makes it possible to protect renal function or reduce the dietary acid load on the kidney.

EP 4 129 279 A1

**Description**

Technical Field

The present invention relates to a novel pharmaceutical composition containing citric acid, a pharmaceutically acceptable salt of citric acid, a hydrate of citric acid, a hydrate of the pharmaceutically acceptable salt of citric acid, or a mixture thereof. More specifically, the present invention relates to a pharmaceutical composition for protecting renal function or reducing a dietary acid load on the kidney, containing citric acid, a pharmaceutically acceptable salt of citric acid, a hydrate of citric acid, a hydrate of the pharmaceutically acceptable salt of citric acid, or a mixture thereof.

Background Art

The number of patients with end-stage kidney disease (ESKD) requiring dialysis or transplantation is increasing worldwide. The number of dialysis patients is increasing also in Japan, and was 320,000 at the end of 2014.

As a preliminary disease of ESKD, chronic kidney disease (CKD) is recognized. CKD is a concept including a kidney disease that continues chronically regardless of a primary disease, and is a concept including all pathological conditions in which there is a decrease in renal function expressed by a glomerular filtration rate (GFR) or findings suggesting kidney disorder are observed persistently and chronically (three months or more). Since CKD is not only a risk of progression to ESKD, but also a strong risk of developing cardiovascular disease (CVD), or the like, it is extremely important to detect CKD at an early-stage and to perform an appropriate treatment. A large number of CKD treatment methods have been established so far, but such methods are still insufficient, and development of a renal protective agent has been demanded.

In a patient with progressed CKD, since the concentration of bicarbonate ions ($HCO_3^-$) in blood is lowered and metabolic acidosis develops, an alkalizing agent such as sodium bicarbonate or a citric acid formulation is administered. Furthermore, it has been reported that progression of CKD is suppressed by administration of sodium bicarbonate which is an alkalizing agent (Non Patent Literature 1). In addition, it has been reported that in an animal model with nephrotic syndrome caused by protein overload, renal tubule cell damage due to aciduria is suppressed by oral administration of sodium bicarbonate (Non Patent Literature 2). In addition, it is also known that administration of an alkalizing agent to an early-stage CKD patient suppresses progression of renal disorder and decreases the concentration of a uremic substance in blood (Patent Literatures 1 and 2).

Meanwhile, it is known that progression of chronic kidney disease is faster since a non-volatile acid load having high dietary dependence is higher. Net acid excretion (NAE) refers to the net amount of acid excreted in urine per unit time. By increasing net acid excretion, it is possible to reduce the dietary acid load on the kidney, particularly to reduce the dietary acid load on the kidney in chronic kidney disease, and it can be expected to suppress progression of chronic kidney disease.

However, it is not known that administration of an alkalizing agent to a CKD patient makes it possible to protect renal function or to reduce the dietary acid load on the kidney.

Citation List

Patent Literature

Patent Literature 1: WO 2018/193648 A
Patent Literature 2: WO 2018/193752 A

Non Patent Literature

Non Patent Literature 1: Brito-Ashurst, I.D., et al.: Bicarbonate supplementation slows progression of CKD and improves nutritional status. J. Am. Soc. Nephrol., 20: 2075-2084, 2009.
Non Patent Literature 2: Souma T., et al.: Luminal alkalinization attenuates proteinuria-induced oxidative damage in proximal tubular cells. J. Am. Soc. Nephrol., 22: 635-648, 2011.

Summary of Invention

Technical Problem

One object of the present invention is to provide a medicine useful for protecting renal function. Another object of the present invention is to provide a medicine useful for reducing the dietary acid load on the kidney. Still another object of the present invention is to provide a food useful for decreasing albumin in spot urine, decreasing albumin in early morning urine, decreasing protein in spot urine, increasing net acid excretion, protecting renal function, or reducing the dietary acid load on the kidney.

Solution to Problem

The present inventors undertook intensive studies for achieving the above objects, and as a result, have found that citric acid, a pharmaceutically acceptable salt of citric acid, a hydrate of citric acid, a hydrate of the pharmaceutically acceptable salt of citric acid, or a mixture thereof, has the action of decreasing albumin in spot urine, the action of decreasing albumin in early morning urine, the action of decreasing protein in spot urine, and the action of increasing net acid excretion, thereby being useful for protecting renal function or reducing the dietary acid load on the kidney, and have completed the present invention.
The present invention has the following aspects.

(1) A pharmaceutical composition for protecting renal function, containing citric acid, a pharmaceutically acceptable salt of citric acid, a hydrate of citric acid, a hydrate of the pharmaceutically acceptable salt of citric acid, or a mixture thereof.

(2) A pharmaceutical composition for reducing the dietary acid load on the kidney, containing citric acid, a pharmaceutically acceptable salt of citric acid, a hydrate of citric acid, a hydrate of the pharmaceutically acceptable salt of citric acid, or a mixture thereof.

(3) The pharmaceutical composition according to (1) or (2), which is administered to an early-stage chronic kidney disease patient.

(4) The pharmaceutical composition according to any one of (1) to (3), which decreases the concentration of protein in urine.

(5) The pharmaceutical composition according to (4), in which the protein in urine is albumin in urine (more specifically, albumin in spot urine or albumin in early morning urine) or protein in spot urine.

(6) The pharmaceutical composition according to (4) or (5), in which the urine is early morning urine.

(7) The pharmaceutical composition according to any one of (1) to (3), which suppresses an increase in concentration of protein in urine associated with progression of chronic kidney disease.

(8) The pharmaceutical composition according to any one of (1) to (7), in which the citric acid, the pharmaceutically acceptable salt of citric acid, the hydrate of citric acid, the hydrate of the pharmaceutically acceptable salt of citric acid, or the mixture thereof, is sodium citrate or a hydrate of sodium citrate, potassium citrate or a hydrate of potassium citrate, or a mixture thereof.

(9) The pharmaceutical composition according to any one of (1) to (8), in which the citric acid, the pharmaceutically acceptable salt of citric acid, the hydrate of citric acid, the hydrate of the pharmaceutically acceptable salt of citric acid, or the mixture thereof, contains a mixture of sodium citrate or a hydrate of sodium citrate and potassium citrate or a hydrate of potassium citrate.

(10) The pharmaceutical composition according to any one of (1) to (9), in which the pharmaceutical composition is in the form of a tablet.

(11) The pharmaceutical composition according to any one of (1) to (10), in which the citric acid, the pharmaceutically acceptable salt of citric acid, the hydrate of citric acid, the hydrate of the pharmaceutically acceptable salt of citric acid, or the mixture thereof, is administered in an amount of 1 to 3 g per day.

(12) The pharmaceutical composition according to any one of (1) to (11), in which the citric acid, the pharmaceutically acceptable salt of citric acid, the hydrate of citric acid, the hydrate of the pharmaceutically acceptable salt of citric acid, or the mixture thereof, is administered in an amount of 1 to 1.5 g per day.

(13) The pharmaceutical composition according to any one of (1) to (12), in which the citric acid, the pharmaceutically acceptable salt of citric acid, the hydrate of citric acid, the hydrate of the pharmaceutically acceptable salt of citric acid, or the mixture thereof, is administered for 12 weeks or more.

(14) The pharmaceutical composition according to any one of (1) to (13), containing anhydrous citric acid.

The present invention also has the following aspects.

(15) A food composition containing citric acid, a pharmaceutically acceptable salt of citric acid, a hydrate of citric acid, a hydrate of the pharmaceutically acceptable salt of citric acid, or a mixture thereof, and used for decreasing albumin in spot urine, decreasing albumin in early morning urine, decreasing protein in spot urine, increasing net acid excretion, protecting renal function, or reducing the dietary acid load on the kidney.

(16) The food composition according to (15), in which the citric acid, the pharmaceutically acceptable salt of citric acid, the hydrate of citric acid, the hydrate of the pharmaceutically acceptable salt of citric acid, or the mixture thereof, contains a mixture of sodium citrate or a hydrate of sodium citrate and potassium citrate or a hydrate of potassium citrate.

(17) The food composition according to (15) or (16), in which the citric acid, the pharmaceutically acceptable salt of citric acid, the hydrate of citric acid, the hydrate of the pharmaceutically acceptable salt of citric acid, or the mixture thereof, is sodium citrate or a hydrate of sodium citrate.

(18) The food composition according to any one of (15) to (17), which is in the form of a tablet.

(19) The food composition according to any one of (15) to (18), in which a packaging, a container, or an instruction of the food composition indicates an effect of decreasing albumin in spot urine, decreasing albumin in early morning urine, decreasing protein in spot urine, increasing net acid excretion, protecting renal function, or reducing the dietary acid load on the kidney.

(20) The food composition according to any one of (15) to (19), which is ingested by a healthy person who is concerned about protein in urine or kidney health.

(21) The food composition according to any one of (15) to (19), which is ingested by a healthy person who is concerned about the dietary acid load on the kidney.

Advantageous Effects of Invention

A pharmaceutical composition, a food composition, or the like, provided by the present invention can decrease albumin in spot urine, decrease albumin in early morning urine, decrease protein in spot urine, and increase net acid excretion in mammals.

The pharmaceutical composition, food composition, or the like, provided by the present invention can protect renal function or reduce the dietary acid load on the kidney in mammals.

Description of Embodiments

1. Pharmaceutical composition

A pharmaceutical composition provided by the present invention can contain, as an active ingredient, citric acid, a pharmaceutically acceptable salt of citric acid, a hydrate of citric acid, a hydrate of the pharmaceutically acceptable salt of citric acid, or a mixture thereof.

Examples of the pharmaceutically acceptable salt of citric acid include an alkali metal salt of citric acid. Examples of the alkali metal salt of citric acid include potassium citrate and sodium citrate, which may be stable hydrates such as a monohydrate of potassium citrate ($C_6H_5K_3O_7 \cdot H_2O$) and a dihydrate of sodium citrate ($C_6H_5Na_3O_7 \cdot 2H_2O$), respectively.

Preferable examples of the active ingredient contained in the pharmaceutical composition provided by the present invention include sodium citrate, potassium citrate, a hydrate of sodium citrate, a hydrate of potassium citrate, and a mixture thereof, and the active ingredient may be, for example, a mixture of a monohydrate of potassium citrate ($C_6H_5K_3O_7 \cdot H_2O$) and a dihydrate of sodium citrate ($C_6H_5Na_3O_7 \cdot 2H_2O$). The mixing ratio between the monohydrate of potassium citrate ($C_6H_5K_3O_7 \cdot H_2O$) and the dihydrate of sodium citrate ($C_6H_5Na_3O_7 \cdot 2H_2O$) can be appropriately set by a person skilled in the art, and for example, the molar ratio between the monohydrate of potassium citrate and the dihydrate of sodium citrate can be set such that the dihydrate of sodium citrate is 0.01 to 100 with respect to 1 of the monohydrate of potassium citrate. The molar ratio between potassium citrate (for example, monohydrate of potassium citrate) and sodium citrate (for example, dihydrate of sodium citrate) can be appropriately set by a person skilled in the art, and may be, for example, 0.85 : 1.15 to 1.15 : 0.85, 0.90 : 1.10 to 1.10 : 0.90, 0.95 : 1.05 to 1.05 : 0.95, or 0.99 : 1.01 to 1.01 : 0.99, and is preferably 1 : 1.

Examples of the active ingredient contained in the pharmaceutical composition provided by the present invention further include sodium citrate or a hydrate thereof, and the active ingredient may be, for example, a dihydrate of sodium citrate ($C_6H_5Na_3O_7 \cdot 2H_2O$).

Examples of the active ingredient contained in the pharmaceutical composition provided by the present invention further include potassium citrate or a hydrate thereof, and the active ingredient may be, for example, a monohydrate of potassium citrate ($C_6H_5K_3O_7 \cdot H_2O$).

In one embodiment, the active ingredient contained in the pharmaceutical composition of the present invention may contain a mixture of sodium citrate or a hydrate thereof and potassium citrate or a hydrate thereof.

In one embodiment, the active ingredient contained in the pharmaceutical composition of the present invention may be a mixture of potassium citrate, sodium citrate, and citric acid (for example, anhydrous citric acid). In this case, the mixing ratio among the citric acid (for example, anhydrous citric acid), the potassium citrate, and the sodium citrate can be appropriately set by a person skilled in the art, and may be, for example, 1 : 1.7 to 2.3 : 1.7 to 2.3, 1 : 1.9 to 2.1 : 1.9 to 2.1, or 1 : 1.95 to 2.05 : 1.95 to 2.05, and is preferably 1 : 2 : 2.

In one embodiment, the active ingredient contained in the pharmaceutical composition of the present invention may be a mixture of a monohydrate of potassium citrate ($C_6H_5K_3O_7 \cdot H_2O$), a dihydrate of sodium citrate ($C_6H_5Na_3O_7 \cdot 2H_2O$), and anhydrous citric acid. In this case, the mixing ratio among the anhydrous citric acid, the monohydrate of potassium citrate ($C_6H_5K_3O_7 \cdot H_2O$), and dihydrate of sodium citrate ($C_6H_5Na_3O_7 \cdot 2H_2O$) can be appropriately set by a person skilled in the art, and may be, for example, 1 : 1.7 to 2.3 : 1.7 to 2.3, 1 : 1.9 to 2.1 : 1.9 to 2.1, or 1 : 1.95 to 2.05 : 1.95 to 2.05, and is preferably 1 : 2 : 2.

In one embodiment, the active ingredient contained in the pharmaceutical composition of the present invention may be formed of only a mixture of sodium citrate or a hydrate thereof and potassium citrate or a hydrate thereof.

When the present specification refers to the weight of citric acid, a pharmaceutically acceptable salt of citric acid, a hydrate of citric acid, a hydrate of the pharmaceutically acceptable salt of citric acid, or a mixture thereof (for example, a monohydrate of potassium citrate ($C_6H_5K_3O_7 \cdot H_2O$) and a dihydrate of sodium citrate ($C_6H_5Na_3O_7 \cdot 2H_2O$)), the weight can be on a dry basis.

In the present specification, an expression [A, B, and/or C] indicates that "at least one selected from the group consisting of A, B, and C".

In one embodiment, administration of a pharmaceutical composition provided by the present invention reduces the dietary acid load on the kidney.

In one embodiment, by administration of a pharmaceutical composition provided by the present invention, an action of protecting renal function and an action of increasing net acid excretion are observed.

In the present specification, "suppression" is a concept including stopping or decelerating exacerbation or progression of a symptom, a condition, or a disease, and performance for this purpose, and including improvement of the symptom, the condition, or the disease, or performance for this purpose. Here, "improvement" is a concept including bringing a "pathological" or "abnormal" symptom, condition, or disease closer to a "healthy" or "normal" condition, or performance for this purpose, and bringing the "pathological" or "abnormal" symptom, condition, or disease to a "healthy" or "normal" condition, or performance for this purpose. Therefore, in one embodiment, "improvement" includes that a numerical value that is an index of a "pathological" or "abnormal" symptom or condition becomes smaller or larger to approach or become a normal value according to the "improvement". The "exacerbation or progression of a symptom, a condition, or a disease" includes exacerbation or progression of a "pathological" or "abnormal" symptom, condition, or disease, and exacerbation or progression from a "healthy" or "normal" condition to a "pathological" or "abnormal" symptom, condition, or disease. In one embodiment, "suppression" is to stop or decelerate exacerbation or progression of a symptom, a condition, or a disease, or performance for this purpose. In another embodiment, suppression" is to stop or decelerate exacerbation or progression of a symptom, a condition, or a disease.

In the present specification, "healthy" refers to absence of acute or chronic disease or disorder, and "normal" indicates that a healthy subject is in a condition to be normally expressed.

Here, the symptom, condition, or disease before administration of the pharmaceutical composition provided by the present invention is compared with that after administration thereof. Alternatively, the symptom, condition, or disease when the pharmaceutical composition provided by the present invention is administered is compared with that when a control or a placebo is administered.

In the present specification, "treatment" is a concept including eliminating, completely recovering, curing, or ameliorating a "pathological" or "abnormal" symptom, condition, or disease, and performance for this purpose, including "suppressing" exacerbation of the "pathological" or "abnormal" symptom, condition, or disease, and performance for this purpose, and also including "improvement". Here, "suppression" and "improvement" have the above meanings. In one embodiment, "treatment" is to eliminate, completely recover, cure, or ameliorate a "pathological" or "abnormal" symptom, condition, or disease, and performance for this purpose. In another embodiment, "treatment" is to eliminate, completely recover, cure, or ameliorate a "pathological" or "abnormal" symptom, condition, or disease.

In the present specification, "prevention" is a concept including preventing onset of a "pathological" or "abnormal" symptom, condition, or disease, and performance for this purpose.

In the present specification, "protection of renal function" is a concept including stopping or decelerating exacerbation of renal function, maintaining the renal function, improving the renal function, or performance for this purpose.

In one embodiment, "protection of renal function" means that exacerbation of renal function after administration of the pharmaceutical composition provided by the present invention is stopped or decelerated, or the renal function is maintained or improved as compared with the renal function before the administration, or that exacerbation of the renal function is stopped or decelerated, or the renal function is maintained or improved by administration of the pharmaceutical composition provided by the present invention as compared with placebo administration or a control.

In one embodiment, "protection of renal function" can be evaluated by the fact that the concentration of albumin in spot urine, the concentration pf albumin in early morning urine, or the concentration of protein in spot urine (concentration; mg/gCr) is decreased by administration of the pharmaceutical composition provided by the present invention as compared with a control.

In one embodiment, "protection of renal function" can be evaluated by the fact that the concentrations of albumin in spot urine (concentration; mg/gCr) 6 weeks, 12 weeks, and 24 weeks after administration of the pharmaceutical composition provided by the present invention are 50% or more and less than 98%, preferably 70% or more and 95% or less, and more preferably 85% or more and 95% or less, with respect to the concentrations of albumin in spot urine 6 weeks, 12 weeks, and 24 weeks after administration of a control, respectively.

In one embodiment, "protection of renal function" can be evaluated by the fact that the concentrations of albumin in early morning urine (concentration; mg/gCr) 6 weeks, 12 weeks, and 24 weeks after administration of the pharmaceutical composition provided by the present invention are 20% or more and less than 95%, preferably 40% or more and 90% or less, and more preferably 60% or more and 90% or less, with respect to the concentrations of albumin in early morning urine 6 weeks, 12 weeks, and 24 weeks after administration of a control, respectively.

In one embodiment, "protection of renal function" can be evaluated by the fact that the concentrations of protein in spot urine (concentration; mg/gCr) 6 weeks, 12 weeks, and 24 weeks after administration of the pharmaceutical composition provided by the present invention are 50% or more and less than 98%, preferably 60% or more and 95% or less, and more preferably 70% or more and 90% or less, with respect to the concentrations of protein in spot urine 6 weeks, 12 weeks, and 24 weeks after administration of a control, respectively.

In one embodiment, "protection of renal function" can be evaluated by the fact that the concentrations of albumin in spot urine (concentration; mg/gCr) 6 weeks, 12 weeks, and 24 weeks after administration of the pharmaceutical composition provided by the present invention are each 60% or more and less than 98%, preferably 70% or more and 95% or less, and more preferably 85% or more and 95% or less, with respect to the concentration of albumin in spot urine before administration.

In one embodiment, "protection of renal function" can be evaluated by the fact that the concentrations of albumin in early morning urine (concentration; mg/gCr) 6 weeks, 12 weeks, and 24 weeks after administration of the pharmaceutical composition provided by the present invention are each 40% or more and less than 95%, preferably 60% or more and 90% or less, and more preferably 70% or more and 90% or less, with respect to the concentration of albumin in early morning urine before administration.

In one embodiment, "protection of renal function" can be evaluated by the fact that the concentrations of protein in spot urine (concentration; mg/gCr) 6 weeks, 12 weeks, and 24 weeks after administration of the pharmaceutical composition provided by the present invention are each 75% or more and less than 98%, preferably 80% or more and 98% or less, and more preferably 85% or more and 95% or less, with respect to the concentration of protein in spot urine before administration.

In one embodiment, "protection of renal function" can be evaluated by the fact that the concentrations of albumin in spot urine (concentration; mg/gCr) 6 weeks, 12 weeks, and 24 weeks after administration of the pharmaceutical composition provided by the present invention are each -60 mg/gCr or more and -3 mg/gCr or less, preferably -50 mg/gCr or more and -5 mg/gCr or less, and more preferably -40 mg/gCr or more and -10 mg/gCr or less as the amount of change with respect to the concentration of albumin in spot urine before administration.

In one embodiment, "protection of renal function" can be evaluated by the fact that the concentrations of albumin in early morning urine (concentration; mg/gCr) 6 weeks, 12 weeks, and 24 weeks after administration of the pharmaceutical composition provided by the present invention are each -60 mg/gCr or more and -5 mg/gCr or less, preferably -50 mg/gCr or more and -5 mg/gCr or less, and more preferably -40 mg/gCr or more and -10 mg/gCr or less as the amount of change with respect to the concentration of albumin in early morning urine before administration.

In one embodiment, "protection of renal function" can be evaluated by the fact that the concentrations of protein in spot urine (concentration; mg/gCr) 6 weeks, 12 weeks, and 24 weeks after administration of the pharmaceutical composition provided by the present invention are each -10 mg/gCr or more and -0.5 mg/gCr or less, preferably -6 mg/gCr or more and -1 mg/gCr or less, and more preferably -5 mg/gCr or more and -1 mg/gCr or less as the amount of change with respect to the concentration of protein in spot urine before administration.

In one embodiment, "reduction of the dietary acid load on the kidney" means that the dietary acid load on the kidney after administration of the pharmaceutical composition provided by the present invention is reduced as compared with the dietary acid load on the kidney before administration, or that administration of the pharmaceutical composition provided by the present invention reduces the dietary acid load on the kidney as compared with placebo administration or control.

In one embodiment, "reduction of the dietary acid load on the kidney" can be evaluated by the fact that the concentration of net acid excretion (concentration; mEq/gCr) is increased by administration of the pharmaceutical composition provided by the present invention as compared with a control.

In one embodiment, "reduction of the dietary acid load on the kidney" can be evaluated by the fact that the concentrations of net acid excretion (concentration; mEq/gCr) 6 weeks, 12 weeks, and 24 weeks after administration of the pharmaceutical composition provided by the present invention are 0.3 mEq/gCr or more and 30 mEq/gCr or less, preferably 0.3mEq/gCr or more and 10 mEq/gCr or less, and more preferably 1 mEq/gCr or more and 10 mEq/gCr or less as the amount of change with respect to the concentrations of net acid excretion 6 weeks, 12 weeks, and 24 weeks after administration of a control, respectively.

In one embodiment, "reduction of the dietary acid load on the kidney" can be evaluated by a fact that the concentrations of net acid excretion (concentration; mEq/gCr) 6 weeks, 12 weeks, and 24 weeks after administration of the pharmaceutical composition provided by the present invention are each 0.3 mEq/gCr or more and 10 mEq/gCr or less, preferably 0.3mEq/gCr or more and 10 mEq/gCr or less, and more preferably 1 mEq/gCr or more and 5 mEq/gCr or less as the amount of change with respect to the concentration of net acid excretion before administration.

In the present specification, "in urine" means, for example, "in early morning urine". In the present specification, "early morning urine" refers to urine collected for the first time after a person wakes up, and "spot urine" refers to any urine other than the "early morning urine".

Due to such characteristics of the pharmaceutical composition provided by the present invention, in one aspect, the pharmaceutical composition provided by the present invention can be used as a pharmaceutical composition for protecting renal function.

In another aspect, the pharmaceutical composition provided by the present invention can be used as a pharmaceutical composition for reducing the dietary acid load on the kidney.

In another aspect, the pharmaceutical composition provided by the present invention can be an agent for decreasing albumin in spot urine, an agent for decreasing albumin in early morning urine, an agent for decreasing protein in spot urine, and an agent for increasing net acid excretion.

The pharmaceutical composition provided by the present invention is administered orally or parenterally to a human or other mammals, and examples of the parenteral administration include intravenous administration, subcutaneous administration, intramuscular administration, intraarticular administration, transmucosal administration, transdermal administration, transnasal administration, rectal administration, intrathecal administration, intraperitoneal administration, and topical administration.

The pharmaceutical composition provided by the present invention may be prepared by using citric acid, a pharmaceutically acceptable salt of citric acid, a hydrate of citric acid, a hydrate of the pharmaceutically acceptable salt of citric acid, or a mixture thereof, as it is or by being mixed with a pharmaceutically acceptable carrier, for example, an excipient (such as lactose, D-mannitol, crystalline cellulose, or glucose), a binder (such as hydroxypropyl cellulose (HPC), gelatin, or polyvinyl pyrrolidone (PVP)), a lubricant (such as magnesium stearate, or talc), a disintegrant (such as starch or carboxymethyl cellulose calcium (CMC-Ca)), a diluent (such as water for injection or saline solution), and other additives (such as a pH adjusting agent, a surfactant, a solubilizer, a preservative, an emulsifier, an isotonizing agent, and a stabilizer) as needed, and can be a formulation such as a tablet, a capsule, a suspension, an injection, or a suppository. For example, in order to form a tablet, citric acid, a pharmaceutically acceptable salt of citric acid, a hydrate of citric acid, a hydrate of the pharmaceutically acceptable salt of citric acid, or a mixture thereof, may be mixed with an excipient (such as lactose, D-mannitol, crystalline cellulose, or glucose), a disintegrant (such as starch or carboxymethyl cellulose calcium (CMC-Ca)), a binder (such as hydroxypropyl cellulose (HPC), gelatin, or polyvinyl pyrrolidone (PVP)), a lubricant (such as magnesium stearate or talc), and the like, and formulated into a tablet.

The tablet according to the present invention will be described in more detail below.

In one embodiment, the pharmaceutical composition provided by the present invention is in the form of a tablet. The tablet provided by the present invention may contain, in addition to an active ingredient (for example, potassium citrate or a hydrate thereof, sodium citrate or a hydrate thereof, or a mixture of a monohydrate of potassium citrate and a dihydrate of sodium citrate), a pharmaceutically acceptable additive that is conventionally used in the field of pharmaceuticals. Examples of such an additive include an excipient, a binder, a disintegrant, a fluidizing agent, a flavoring agent, a lubricant, a pH adjusting agent, a surfactant, a stabilizer, and a flavor.

The content of the active ingredient in the tablet provided by the present invention may be 10 to 95% by weight, preferably 30 to 90% by weight, and more preferably 60 to 85% by weight, with respect to the tablet.

Examples of the excipient that can be used in the tablet provided by the present invention include a saccharide such as lactose (for example, lactose hydrate or anhydrous lactose), glucose, sucrose, fructose, or maltose, a sugar alcohol such as erythritol, sorbitol, maltitol, xylitol, or D-mannitol, starch (for example, corn starch, potato starch, rice starch, or wheat starch), crystalline cellulose, magnesium aluminometasilicate, anhydrous calcium phosphate, precipitated calcium carbonate, calcium silicate, calcium lactate, and ethyl cellulose, and crystalline cellulose is particularly preferable.

The content of the excipient in the tablet provided by the present invention may be 1 to 95% by weight, preferably 1 to 80% by weight, more preferably 3 to 80% by weight, and still more preferably 3 to 20% by weight, with respect to the tablet.

Examples of the binder that can be used in the tablet provided by the present invention include hydroxypropyl cellulose, hydroxypropyl methylcellulose, polyvinylpyrrolidone, dextrin, methylcellulose, polyvinyl alcohol, sodium alginate, an aminoalkyl methacrylate copolymer, polyethylene glycol, pregelatinized starch (for example, partially pregelatinized starch), agar, and gelatin, and hydroxypropyl cellulose is particularly preferable.

The content of the binder in the tablet provided by the invention may be 0.1 to 30% by weight, preferably 0.1 to 10% by weight, and more preferably 0.3 to 3% by weight, with respect to the tablet.

Examples of the disintegrant that can be used in the tablet provided by the present invention include croscarmellose sodium, carmellose calcium, sodium carboxymethyl starch, hydroxypropyl cellulose with low degree of substitution, crospovidone, starch (for example, wheat starch, corn starch, or partially pregelatinized starch), and carmellose, and partially pregelatinized starch is particularly preferable.

The content of the disintegrant in the tablet provided by the present invention may be 0.3 to 20% by weight, preferably 1 to 10% by weight, and more preferably 3 to 10% by weight, with respect to the tablet.

Examples of the fluidizing agent that can be used in the tablet provided by the present invention include light anhydrous silicic acid, talc, and magnesium aluminate metasilicate.

The content of the fluidizing agent in the tablet provided by the present invention may be 0.03 to 3% by weight, preferably 0.1 to 3% by weight, and more preferably 0.3 to 3% by weight, with respect to the tablet.

Examples of the flavoring agent that can be used in the tablet provided by the present invention include an acidulant such as citric acid (for example, anhydrous citric acid), malic acid, acetic acid, tartaric acid, fumaric acid, or ascorbic acid (note that the flavoring agent is not included in the active ingredient according to the present invention), and a sweetener such as sodium saccharin, dipotassium glycyrrhizinate, aspartame (registered trademark), stevia, thaumatin, or sucralose.

The content of the flavoring agent in the tablet provided by the invention may be 0.03 to 3% by weight, preferably 0.1 to 3% by weight, and more preferably 0.3 to 3% by weight, with respect to the tablet.

Examples of the lubricant that can be used in the tablet provided by the present invention include magnesium stearate, calcium stearate, talc, light anhydrous silicic acid, a sucrose fatty acid ester, carnauba wax, macrogol, and sodium stearyl fumarate, and magnesium stearate is particularly preferable.

The content of the lubricant in the tablet provided by the present invention may be 0.1 to 30% by weight, preferably 0.3 to 10% by weight, and more preferably 1 to 3% by weight, with respect to the tablet.

Examples of the pH adjusting agent that can be used in the tablet provided by the present invention include citric acid, a phosphate (for example, sodium dihydrogen phosphate or potassium dihydrogen phosphate), a carbonate (for example, magnesium carbonate or sodium carbonate), a tartrate, a fumarate, an acetate, and an amino acid salt (note that the pH adjusting agent is not included in the active ingredient according to the present invention).

The content of the pH adjusting agent in the tablet provided by the present invention may be 0.1 to 30% by weight, preferably 0.3 to 10% by weight, and more preferably 1 to 5% by weight, with respect to the tablet.

Examples of the surfactant that can be used in the tablet provided by the present invention include sodium lauryl sulfate, polysorbate, a sucrose fatty acid ester, polyoxyethylene-hydrogenated castor oil, polyoxyl stearate, macrogol, and a poloxamer.

The content of the surfactant in the tablet provided by the present invention may be 0.01 to 3% by weight, preferably 0.03 to 1% by weight, and more preferably 0.03 to 0.5% by weight, with respect to the tablet.

Examples of the stabilizers that can be used in the tablet provided by the present invention include citric acid (for example, anhydrous citric acid), malic acid, acetic acid, tartaric acid, maleic acid, ascorbic acid, sodium edetate, and tocopherol (note that the stabilizer is not included in the active ingredient according to the present invention), and anhydrous citric acid is particularly preferable.

The content of the stabilizer in the tablet provided by the invention may be 0.01 to 30% by weight, preferably 0.1 to 30% by weight, and more preferably 1 to 20% by weight, with respect to the tablet.

Examples of the flavor that can be used in the tablet provided by the present invention include a citrus flavor such as lemon, orange, or grapefruit, peppermint, spearmint, and menthol, and the flavor can be contained in the tablet in an appropriate amount (for example, 0.01 to 1% by weight, more preferably 0.01 to 0.1% by weight, with respect to the tablet).

The total content of the active ingredients and the pharmaceutically acceptable additives in the tablet provided by the present invention does not exceed 100% by weight with respect to the tablet.

The tablet provided by the present invention can be an uncoated tablet containing the above components and not coated with a coating layer, or can be a film-coated tablet coated with a coating layer. The content of the coating layer can be appropriately set by a person skilled in the art, and may be, for example, 0.1 to 10% by weight with respect to the uncoated tablet. The coating layer can appropriately contain a plasticizer, a colorant, a brightener, and the like in addition to a coating base.

Examples of the coating base that can be used in the tablet provided by the present invention include hydroxypropyl cellulose, hydroxypropyl methyl cellulose, ethyl cellulose, cellulose acetate phthalate, a methacrylic acid copolymer, and polyvinylpyrrolidone, and hydroxypropyl methyl cellulose is particularly preferable. The content of the coating base in the tablet provided by the present invention may be 0.01 to 10% by weight, and preferably 0.3 to 3% by weight, with respect to the tablet.

Examples of the coating plasticizer that can be used in the tablet provided by the present invention include triethyl citrate, medium chain fatty acid triglycerides, triacetin, glycerin, propylene glycol, and polyethylene glycol (for example, macrogol 6000), and macrogol 6000 is particularly preferable. The content of the coating plasticizer in the tablet provided by the present invention may be 0.01 to 1% by weight, and preferably 0.03 to 3% by weight, with respect to the tablet.

Examples of the coating colorant that can be used in the tablet provided by the present invention include titanium oxide, yellow iron sesquioxide, iron sesquioxide, black iron oxide, edible blue No. 2, and edible blue No. 2 aluminum lake. The content of the coating colorant in the tablet provided by the present invention may be 0.01 to 1% by weight, and preferably 0.03 to 3% by weight, with respect to the tablet.

Examples of the coating brightener that can be used in the tablet provided by the present invention include carnauba wax. The content of the coating brightener in the tablet provided by the invention may be 0.0001 to 0.1% by weight, and preferably 0.001 to 0.01% by weight, with respect to the tablet.

The pharmaceutical composition provided by the present invention can be produced by a method known in the field of pharmaceuticals. For example, when the pharmaceutical composition is formed into a tablet, a production method thereof can include a mixing step of mixing an active ingredient (for example, potassium citrate or a hydrate thereof, sodium citrate or a hydrate thereof, or a mixture of a monohydrate of potassium citrate and a dihydrate of sodium citrate) with an additive, a granulation step, a tableting step, and/or a coating step.

The mixing step can include a step of mixing the active ingredient with an additive such as an excipient, a stabilizer, a disintegrant, and/or a binder. The method can further include a step of mixing the mixture containing the active ingredient and the additive with a lubricant, a flavoring agent, and/or a flavor before the tableting step. Mixing can be performed using a V-type mixer, a W-type mixer, a container mixer, a tumbler mixer, a stirring mixer, or the like.

The granulation step can be performed by a granulation method known in the field of pharmaceuticals. Examples of the granulation method include a dry granulation method, a wet granulation method, and a fluidized bed granulation method.

As one embodiment, the mixture obtained in the mixing step and the granulated product obtained in the granulation step are appropriately pulverized and/or sieved, and can be thereby formed into a mixture and a granulated product having desired particle diameters, respectively. The pulverization can be performed by, for example, a pulverizer known in the field of pharmaceuticals, such as a ball mill, a jet mill, or a hammer mill. The sieving can be performed using a 16 mesh sieve (opening: 1000 um) to a 32 mesh sieve (opening: 500 $\mu$m), or the like.

The tableting step can be performed by a tableting method known in the field of pharmaceuticals. Examples of the tableting method include a direct tableting method, a dry tableting method, a wet tableting method, and an external lubricating tableting method. For example, the mixture and the granulated product obtained in the above steps can be tableted using a tableting machine known in the field of pharmaceuticals, such as a single-stroke tableting machine or a rotary tableting machine. When the single-stroke tableting machine, the rotary tableting machine, or the like is used, a tableting pressure of 1 kN to 30 kN can be adopted.

The coating step can be performed by a method known in the field of pharmaceuticals. For example, the coating can be performed by spray-coating an outer side of an uncoated tablet with a coating liquid appropriately containing a coating base, a plasticizer, a colorant, a brightener, and the like.

In one embodiment, the tablet provided by the present invention can be produced by mixing an active ingredient, an excipient (for example, lactose, D-mannitol, crystalline cellulose, and/or glucose), a binder (for example, hydroxypropyl cellulose (HPC), gelatin, and/or polyvinylpyrrolidone (PVP)), a stabilizer (for example, anhydrous citric acid), a disintegrant (for example, starch (for example, partially pregelatinized starch) and/or carboxymethylcellulose calcium (CMC-Ca)), and a lubricant (for example, magnesium stearate), and tableting the mixture to obtain an uncoated tablet, and forming a coating layer containing a coating base (for example, hydroxypropyl cellulose, hydroxypropyl methylcellulose, and/or PVP), a plasticizer (for example, triethyl citrate and/or macrogol 6000), a colorant (for example, iron sesquioxide and/or titanium oxide), and a brightener (for example, carnauba wax) on an outer side of the uncoated tablet.

In one embodiment, the hardness of a tablet to be obtained can be 10 to 200 N, and preferably 30 to 150 N.

The content of the active ingredient in the pharmaceutical composition provided by the present invention can be appropriately set.

In one embodiment, the content of the active ingredient in the pharmaceutical composition provided by the present invention may be set such that a dose of the active ingredient is equal to or smaller than the amount to improve acidic urine in gout or hyperuricemia by being administered to a human, for example, such that the dose of the active ingredient is 1 to 50% or 10 to 20% of a daily dose approved in Japan for improving acidic urine in gout or hyperuricemia (for example, in the case where the active ingredient is a citric acid formulation: two tablets each containing 231.5 mg of potassium citrate ($C_6H_5K_3O_7{\cdot}H_2O$) and 195.0 mg of sodium citrate hydrate ($C_6H_5Na_3O_7{\cdot}2H_2O$) are orally administered three times a day).

In one embodiment, the pharmaceutical composition provided by the present invention is a tablet, and a monohydrate of potassium citrate or a dihydrate of sodium citrate as the active ingredient may be contained in one tablet in an amount of 10 mg to 1 g, preferably 100 mg to 500 mg, more preferably 400 mg to 500 mg.

In one embodiment, the pharmaceutical composition provided by the present invention is a tablet, and a monohydrate of potassium citrate and a dihydrate of sodium citrate may be contained in one tablet each in an amount of 10 mg to 300 mg and in a total amount of 20 mg to 600 mg, preferably each in an amount of 150 to 250 mg and in a total amount of 400 to 500 mg, more preferably each in an amount of 190 to 240 mg and in a total amount of 400 to 450 mg.

As one embodiment, the pharmaceutical composition provided by the present invention is in a form of a tablet, may contain 231.5 mg of a monohydrate of potassium citrate and 195.0 mg of a dihydrate of sodium citrate as active ingredients, and may contain anhydrous citric acid, crystalline cellulose, partially pregelatinized starch, hydroxypropyl cellulose, magnesium stearate, hypromellose, macrogol 6000, titanium oxide, and carnauba wax as additives.

As one embodiment, a tablet containing 231.5 mg of a monohydrate of potassium citrate and 195.0 mg of a dihydrate of sodium citrate may be used as one administration unit.

In the present specification, "administration unit" indicates a unit of a formulation, and "one administration unit" indicates a minimum unit of a formulation. Therefore, for example, in a case of a tablet, the administration unit is each tablet, and one administration unit refers to one tablet. In the case of an injection product, the administration unit is an injection product contained in a sealed container such as an ampule or a vial, and one administration unit refers to an injection product contained in one sealed container such as an ampule or a vial.

In the case where the pharmaceutical composition provided by the present invention is administered to a human or another mammal, one or more administration units may be administered at a time, or an administration unit may be divided to be administered.

The dose of the active ingredient is appropriately determined according to the kind of active ingredient, the administering method, the age, body weight, sex, symptom, and sensitivity to a drug of an administering subject, and the like, but may be adjusted according to status of improvement in symptom.

In one embodiment, the dose of the active ingredient may be such that the pH of human urine (for example, early morning urine) is pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to pH 6.8, pH 6.2 to pH 6.5, or pH 6.5 to pH 6.8, by oral administration of the active ingredient.

In one embodiment, the dose of the active ingredient may be such that the pH of human urine (for example, early morning urine) is pH 5.2 to pH 6.8, pH 5.5 to pH 6.8, pH 5.8 to pH 6.8, pH 5.8 to pH 6.5, pH 5.8 to pH 6.2, pH 5.8 or more and less than pH 6.2, pH 6.0 to pH 6.5, pH 6.0 to pH 6.4, pH 6.0 to pH 6.3, pH 6.0 to pH 6.2, pH 6.0 or more and less than pH 6.2, pH 6.1 to pH 6.3, pH 6.2 to pH 6.8, pH 6.2 to pH 6.5, or pH 6.5 to pH 6.8, by oral administration of the active ingredient 6 weeks, 12 weeks, or 24 weeks after administration.

In one embodiment, when a mixture of a monohydrate of potassium citrate and a dihydrate of sodium citrate as an active ingredient is orally administered to a human, the monohydrate of potassium citrate and the dihydrate of sodium citrate may be administered each in an amount of 0.1 to 5 g/day and in a total of 0.2 to 10 g/day, each in an amount of 0.1 to 3 g/day and in a total of 0.2 to 6 g/day, or each in an amount of 0.5 to 3 g/day and in a total of 1 to 6 g/day, preferably each in an amount of 0.5 to 1.5 g/day and in a total of 1 to 3 g/day, each in an amount of 1 to 1.5 g/day and in a total of 2 to 3 g/day, or each in an amount of 0.5 to 1 g/day and in a total of 1 to 2 g/day, while the daily dose is divided into one to five portions, preferably three portions.

In one embodiment, when a monohydrate of potassium citrate or a dihydrate of sodium citrate as an active ingredient is orally administered to a human, a monohydrate of potassium citrate or a dihydrate of sodium citrate may be administered in an amount of 1 to 10 g/day, 1 to 6 g/day, 2 to 5.5 g/day, 1 to 3 g/day, 2 to 3 g/day, or 1 to 1.5 g/day, while the daily dose is divided into one to five portions, preferably three portions.

In one embodiment, citric acid, a pharmaceutically acceptable salt of citric acid, a hydrate of citric acid, a hydrate of the pharmaceutically acceptable salt of citric acid, or a mixture thereof may be administered over a long period of time, and is administered, for example, for 1 week, 2 weeks, 3 weeks, 6 weeks, 8 weeks, 10 weeks, 12 weeks, 24 weeks, 40 weeks, 60 weeks, 80 weeks, 100 weeks, 120 weeks, 1 week or more, 2 weeks or more, 3 weeks or more, 6 weeks or more, 8 weeks or more, 10 weeks or more, 12 weeks or more, 24 weeks or more, 40 weeks or more, 60 weeks or more, 80 weeks or more, 100 weeks or more, 120 weeks or more, 6 weeks or more and 24 weeks or less, 12 weeks or more and 24 weeks or less, 6 weeks or more and 30 weeks or less, 12 weeks or more and 30 weeks or less, 6 weeks or more and 40 weeks or less, 12 weeks or more and 40 weeks or less, 6 weeks or more and 60 weeks or less, 12 weeks or more and 60 weeks or less, 6 weeks or more and 80 weeks or less, 12 weeks or more and 80 weeks or less, 6 weeks or more and 100 weeks or less, 12 weeks or more and 100 weeks or less, 6 weeks or more and 120 weeks or less, or 12 weeks or more and 120 weeks or less.

In one embodiment, by continuously administering the pharmaceutical composition provided by the present invention for 6 weeks, 12 weeks, and/or 24 weeks, an effect (for example, an effect of decreasing albumin in spot urine, an effect of decreasing albumin in early morning urine, an effect of decreasing protein in spot urine, an effect of increasing net acid excretion, an effect of protecting renal function, or an effect of reducing the dietary acid load on the kidney) beneficial to a patient with kidney disease (for example, diabetic nephropathy or early-stage chronic kidney disease with diabetes) can be detected.

As one embodiment, the pharmaceutical composition provided by the present invention is administered to a human suffering from kidney disease. The kidney disease includes an acute kidney disease and a chronic kidney disease, unless otherwise noted.

Examples of the acute kidney disease include an acute kidney disease caused by a drug (for example, a non-steroidal anti-inflammatory drug, an angiotensin converting enzyme inhibitor, an angiotensin II receptor blocker, an aminoglycoside-based antibiotic, a new quinolone-based antibacterial agent, an iodine contrast agent, or a platinum formulation such as cisplatin) and an acute kidney disease caused by renal ischemia.

The chronic kidney disease (CKD) is a concept including a kidney disease that continues chronically regardless of a primary disease, and is a concept including all pathological conditions in which there is a decrease in renal function expressed by a glomerular filtration rate (GFR) or findings suggesting kidney disorder are observed persistently and chronically (three months or more).

As one embodiment, the pharmaceutical composition provided by the present invention is administered to a human suffering from diabetic nephropathy, particularly diabetic nephropathy which is an early-stage chronic kidney disease.

According to CKD Clinical Guide 2012 (Journal of the Japanese Society of Nephrology 2012), the severity of chronic kidney disease is evaluated by classification with cause (Cause: C), renal function (GFR: G), and proteinuria (albuminuria: A).

Categories by GFR are as follows.

G1: GFR is normal or high ($\geq$ 90 mL/min/1.73 m$^2$)
G2: GFR is normal or mildly decreased (60 to 89 mL/min/1.73 m$^2$)
G3a: GFR is mildly to moderately decreased (45 to 59 mL/min/1.73 m$^2$)
G3b: GFR is moderately to severely decreased (30 to 44 mL/min/1.73 m$^2$)
G4: GFR is severely decreased (15 to 29 mL/min/1.73 m2)
G5: end-stage kidney disease (ESKD) (< 15 mL/min/1.73 m$^2$)

In the case where the primary disease is diabetes, categories by proteinuria (albuminuria: A) are classified as follows using a urinary albumin/creatinine (Cr) ratio.

A1: normal (less than 30 mg/gCr)
A2: microalbuminuria (30 to 299 mg/gCr)
A3: macroalbuminuria (300 mg/gCr or more)

In the case where the primary disease is hypertension, nephritis, polycystic kidney disease, transplanted kidney, or the like other than diabetes, the categories by proteinuria (albuminuria: A) are classified as follows by using a urine protein/creatinine (Cr) ratio.

A1: normal (less than 0.15 g/gCr)
A2: mild proteinuria (0.15 to 0.49 g/gCr)
A3: severe proteinuria (0.50 g/gCr or more)

According to CKD Clinical Guide 2012 (Journal of the Japanese Society of Nephrology 2012), the severity classification of chronic kidney disease (CKD) is expressed as, for example, diabetes G2A3 or chronic nephritis G3bA1 using the above alphabets C, G, and A.

However, the severity of chronic kidney disease has conventionally been expressed only by a stage classified by GFR, and in consideration of this circumstance, the severity of chronic kidney disease can also be expressed by the stages of G1, G2, G3a, G3b, G4, and G5 as in the past.

In one embodiment, the pharmaceutical composition provided by the present invention is administered to an early-stage chronic kidney disease patient with a low severity, for example, a diabetic nephropathy patient.

In one embodiment, the pharmaceutical composition provided by the present invention is administered to a patient with chronic kidney disease of stage G3b or less, preferably stage G2 or less, for example, a diabetic nephropathy patient.

In one embodiment, the pharmaceutical composition provided by the present invention is administered to a patient with chronic kidney disease of stage G2 or more and G3b or less (for example, stage G2 and stage G3a; or stage G2, stage G3a, and stage G3b), for example, a diabetic nephropathy patient.

In one embodiment, the pharmaceutical composition provided by the present invention is administered to a patient with chronic kidney disease of stage G3b or less and microalbuminuria, and preferably to a patient with chronic kidney disease of stage G2 and microalbuminuria, for example, a diabetic nephropathy patient.

In one embodiment, the pharmaceutical composition provided by the present invention is administered to a patient with chronic kidney disease of stage G2 or more and G3b or less (for example, stage G2 and stage G3a; or stage G2, stage G3a, and stage G3b) and microalbuminuria, for example, a diabetic nephropathy patient.

In one embodiment, the pharmaceutical composition provided by the present invention is administered to a patient with chronic kidney disease of stage G3b or less having a protein in urine excretion of less than 3.5 g/gCr, for example, a diabetic nephropathy patient, and preferably to a patient with chronic kidney disease of stage G2 having a protein in urine excretion of less than 3.5 g/gCr, for example, a diabetic nephropathy patient.

In one embodiment, the pharmaceutical composition provided by the present invention is administered to a patient with chronic kidney disease of stage G2 or more and G3b or less (for example, stage G2 and stage G3a; or stage G2, stage G3a, and stage G3b) having a protein in urine excretion of less than 3.5 g/gCr, for example, a diabetic nephropathy patient.

In one embodiment, the pharmaceutical composition provided by the present invention is administered to a patient with progressive chronic kidney disease, for example, a diabetic nephropathy patient.

In one embodiment, the pharmaceutical composition provided by the present invention is administered to a patient who receives treatment according to CKD Clinical Guide. For example, the pharmaceutical composition provided by the present invention is administered to a patient who undergoes blood pressure control (for example, administration of an RA-based inhibitor such as ARB or an ACE inhibitor, a diuretic, or a Ca antagonist), measures against proteinuria (for example, administration of an RA-based inhibitor), blood glucose level control (for example, administration of an $\alpha$-glucosidase inhibitor), lipid control (for example, administration of statin or fibrate), anemia control (for example, administration of erythropoetin), and/or measures against bone and minerals (for example, administration of bisphosphonate) according to CKD Clinical Guide.

In one embodiment, the pharmaceutical composition provided by the present invention is used in combination with an antihypertensive agent (for example an ARB, an ACE inhibitor, a diuretic, or a Ca antagonist).

In one embodiment, the pharmaceutical composition provided by the present invention is used in combination with spherical adsorbed carbon obtained by oxidizing and reducing spherical fine porous carbon derived from a petroleum-based hydrocarbon at a high temperature (sold as Kremezin (registered trademark) in Japan).

In one embodiment, the pharmaceutical composition provided by the present invention is administered to an early-stage chronic kidney disease patient with a low severity (for example, a patient with chronic kidney disease of stage G3b or less, preferably G2 or more and stage G3b or less, more preferably stage G2 and stage G3a, still more preferably stage G2), for example, a diabetic nephropathy patient, and exhibits the action of decreasing albumin in spot urine, the action of decreasing albumin in early morning urine, the action of decreasing protein in spot urine, the action of increasing net acid excretion, the action of protecting renal function, or the action of reducing a dietary acid load on the kidney in the patient. In the present embodiment, the pharmaceutical composition provided by the present invention can be a pharmaceutical composition for protecting renal function or a pharmaceutical composition for reducing the dietary acid load on the kidney.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in protecting renal function, in which a monohydrate of potassium citrate and a dihydrate of sodium citrate are orally administered each in an amount of 0.5 to 1.5 g/day and in a total of 1 to 3 g/day, while the daily dose is divided into one to five portions, preferably three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in protecting renal function, in which one administration unit (preferably one tablet) contains 231.5 mg of a monohydrate of potassium citrate and 195.0 mg of a dihydrate of sodium citrate, and three to six administration units are orally administered per day, while the daily dose is divided into three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in reducing a dietary acid load on the kidney, in which a monohydrate of potassium citrate and a dihydrate of sodium citrate are orally administered each in an amount of 0.5 to 1.5 g/day and in a total of 1 to 3 g/day, while the daily dose is divided into one to five portions, preferably three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in reducing a dietary acid load on the kidney, in which one administration unit (preferably one tablet) contains 231.5 mg of a monohydrate of potassium citrate and 195.0 mg of a dihydrate of sodium citrate, and three to six administration units are orally administered per day, while the daily dose is divided into three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing albumin in spot urine, decreasing albumin in early morning urine, decreasing protein in spot urine, or increasing net acid excretion, in which a monohydrate of potassium citrate and a dihydrate of sodium citrate are orally administered each in an amount of 0.5 to 1.5 g/day and in a total of 1 to 3 g/day, while the daily dose is divided into one to five portions, preferably three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing albumin in spot urine, decreasing albumin in early morning urine, decreasing protein in spot urine, or increasing net acid excretion, in which one administration unit (preferably one tablet) contains 231.5 mg of a monohydrate of potassium citrate and 195.0 mg of a dihydrate of sodium citrate, and three to six administration units are orally administered per day, while the daily dose is divided into three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing albumin in spot urine, decreasing albumin in early morning urine, decreasing protein in spot urine, or increasing net acid excretion in an early-stage chronic kidney disease patient, for example, a diabetic nephropathy patient, in which a monohydrate of potassium citrate and a dihydrate of sodium citrate are orally administered each in an amount of 0.5 to 1.5 g/day and in a total of 1 to 3 g/day, while the daily dose is divided into one to five portions, preferably three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing albumin in spot urine, decreasing albumin in early morning urine, decreasing protein in spot urine, or increasing net acid excretion in an early-stage chronic kidney disease patient, for example, a diabetic nephropathy patient, in which one administration unit (preferably one tablet) contains 231.5 mg of a monohydrate of potassium citrate and 195.0 mg of a dihydrate of sodium citrate, and three to six administration units are orally administered per day, while the daily dose is divided into three portions.

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing the concentration of protein in urine or suppressing an increase in concentration of protein in urine associated with progression of chronic kidney disease, in which a monohydrate of potassium citrate and a dihydrate of sodium citrate are orally administered each in an amount of 0.5 to 1.5 g/day and in a total of 1 to 3 g/day, while the daily dose is divided into one to five portions, preferably three portions. Here, "urine" in the term "in urine" can be "early morning urine". The term "protein in urine" can be "albumin in urine".

In one embodiment, the pharmaceutical composition provided by the present invention is a pharmaceutical composition containing, as an active ingredient, citric acid, a pharmaceutically acceptable salt of citric acid, a hydrate of citric acid, a hydrate of the pharmaceutically acceptable salt of citric acid, or a mixture thereof for use in decreasing the concentration of protein in urine or suppressing an increase in concentration of protein in urine associated with progression of chronic kidney disease, in which one administration unit (preferably one tablet) contains 231.5 mg of a monohydrate of potassium citrate and 195.0 mg of a dihydrate of sodium citrate, and three to six administration units are orally administered per day, while the daily dose is divided into three portions. Here, "urine" in the term "in urine" can be "early morning urine". The term "protein in urine" can be "albumin in urine".

Examples of another embodiment of the present invention include the following ones:

<1-1> A method for protecting renal function in a mammalian subject (for example, human), the method including administering, to a subject in need of renal function protection, an effective amount of citric acid, a pharmaceutically acceptable salt of citric acid, a hydrate of citric acid, a hydrate of the pharmaceutically acceptable salt of citric acid, or a mixture thereof;

<1-2> A method for reducing the dietary acid load on the kidney in a mammalian subject (for example, human), the method including administering, to a subject in need of reduction in dietary acid load on the kidney, an effective amount of citric acid, a pharmaceutically acceptable salt of citric acid, a hydrate of citric acid, a hydrate of the pharmaceutically acceptable salt of citric acid, or a mixture thereof;

<1-3> A method for decreasing albumin in spot urine, decreasing albumin in early morning urine, decreasing protein in spot urine, or increasing net acid excretion in a mammalian subject (for example, human), the method including administering, to a subject in need of decreasing albumin in spot urine, decreasing albumin in early morning urine, decreasing protein in spot urine, or increasing net acid excretion, an effective amount of citric acid, a pharmaceutically acceptable salt of citric acid, a hydrate of citric acid, a hydrate of the pharmaceutically acceptable salt of citric acid, or a mixture thereof;

<2-1> Citric acid, a pharmaceutically acceptable salt of citric acid, a hydrate of citric acid, a hydrate of the pharmaceutically acceptable salt of citric acid, or a mixture thereof, for use in protecting renal function;

<2-2> Citric acid, a pharmaceutically acceptable salt of citric acid, a hydrate of citric acid, a hydrate of the pharmaceutically acceptable salt of citric acid, or a mixture thereof, for use in reducing a dietary acid load on the kidney;

<2-3> Citric acid, a pharmaceutically acceptable salt of citric acid, a hydrate of citric acid, a hydrate of the pharmaceutically acceptable salt of citric acid, or a mixture thereof, for use in decreasing albumin in spot urine, decreasing albumin in early morning urine, decreasing protein in spot urine, or increasing net acid excretion;

<3-1> A pharmaceutical composition containing citric acid, a pharmaceutically acceptable salt of citric acid, a hydrate of citric acid, a hydrate of the pharmaceutically acceptable salt of citric acid, or a mixture thereof, for use in protecting renal function;

<3-2> A pharmaceutical composition containing citric acid, a pharmaceutically acceptable salt of citric acid, a hydrate of citric acid, a hydrate of the pharmaceutically acceptable salt of citric acid, or a mixture thereof, for use in reducing the dietary acid load on the kidney;

<3-3> A pharmaceutical composition containing citric acid, a pharmaceutically acceptable salt of citric acid, a hydrate of citric acid, a hydrate of the pharmaceutically acceptable salt of citric acid, or a mixture thereof, for use in decreasing albumin in spot urine, decreasing albumin in early morning urine, decreasing protein in spot urine, or increasing net acid excretion;

<4-1> Use of citric acid, a pharmaceutically acceptable salt of citric acid, a hydrate of citric acid, a hydrate of the pharmaceutically acceptable salt of citric acid, or a mixture thereof, for producing a pharmaceutical composition for protecting renal function;

<4-2> Use of citric acid, a pharmaceutically acceptable salt of citric acid, a hydrate of citric acid, a hydrate of the pharmaceutically acceptable salt of citric acid, or a mixture thereof, for producing a pharmaceutical composition for reducing the dietary acid load on the kidney; and

<4-3> Use of citric acid, a pharmaceutically acceptable salt of citric acid, a hydrate of citric acid, a hydrate of the pharmaceutically acceptable salt of citric acid, or a mixture thereof, for producing a pharmaceutical composition for decreasing albumin in spot urine, decreasing albumin in early morning urine, decreasing protein in spot urine, or increasing net acid excretion.

2. Food composition

In one embodiment, a food composition provided by the present invention contains citric acid, a pharmaceutically acceptable salt of citric acid, a hydrate of citric acid, a hydrate of the pharmaceutically acceptable salt of citric acid, or a mixture thereof, and exhibits the effect of decreasing albumin in spot urine, decreasing albumin in early morning urine, decreasing protein in spot urine, increasing net acid excretion, protecting renal function, or reducing the dietary acid load on the kidney.

To the citric acid, the pharmaceutically acceptable salt of citric acid, the hydrate of citric acid, the hydrate of the pharmaceutically acceptable salt of citric acid, or the mixture thereof, the citric acid, the pharmaceutically acceptable salt of citric acid, the hydrate of citric acid, the hydrate of the pharmaceutically acceptable salt of citric acid, or the mixture thereof described in "1. Pharmaceutical composition" above can be applied. Examples of the citric acid, the pharmaceutically acceptable salt of citric acid, the hydrate of citric acid, the hydrate of the pharmaceutically acceptable salt of citric acid, or the mixture thereof, include a pharmaceutically acceptable salt of citric acid (for example, an alkali metal citrate, a hydrate of the alkali metal citrate, or a mixture thereof) as a food-acceptable salt of citric acid, and a mixture of a monohydrate of potassium citrate ($C_6H_5K_3O_7 \cdot H_2O$) and a dihydrate of sodium citrate ($C_6H_5Na_3O_7 \cdot 2H_2O$), or a dihydrate of sodium citrate is preferable.

The content of citric acid, a pharmaceutically acceptable salt of citric acid, a hydrate of citric acid, a hydrate of the pharmaceutically acceptable salt of citric acid, or a mixture thereof, in the food composition provided by the present invention can be appropriately determined depending on the kind of food. Examples of the food composition include a food for specified health use, a functionally labeled food, a food for hospital patients, and a supplement. The form of such a food composition is not particularly limited as long as the food composition contains citric acid, a pharmaceutically acceptable salt of citric acid, a hydrate of citric acid, a hydrate of the pharmaceutically acceptable salt of citric acid, or a mixture thereof, in an effective amount for exhibiting the effects described above, and is in an orally ingestible form, and the food composition may be in a form of ordinary food and drink, or may be provided as a formulation suitable for oral administration, for example, a formulation such as a tablet, a capsule, or a suspension, among the formulations that can be applied to the pharmaceutical composition. As to the formulation and production method of such a formulation, in the present specification, the formulation and production method of a pharmaceutical formulation described in "1. Pharmaceutical composition" above can be applied as they are and, further, a formulation technique known per se in the technical field of pharmaceutical formulation can be applied.

For example, a food for specified health use, a functionally labeled food, a food for hospital patients, or a supplement may contain a monohydrate of potassium citrate and a dihydrate of sodium citrate as active ingredients in total in an amount of 1/3 of 1 to 3 g per serving of food. In the case where a food for specified health use, a functionally labeled food, a food for hospital patients, or a supplement is provided as a tablet, such a food or a supplement may contain, for example, citric acid, a pharmaceutically acceptable salt of citric acid, a hydrate of citric acid, a hydrate of the pharmaceutically acceptable salt of citric acid, or a mixture thereof, in an amount of 70 to 80% by weight per tablet of 300 mg to 600 mg.

When the food composition provided by the present invention is not formulated and is provided in a form of ordinary food or drink, the food composition can be appropriately produced by a person skilled in the art depending on the kind of the food and, for example, can be produced by blending citric acid, a pharmaceutically acceptable salt of citric acid, a hydrate of citric acid, a hydrate of the pharmaceutically acceptable salt of citric acid, or a mixture thereof (for example, potassium citrate and/or sodium citrate), with a food material.

Examples of the form of the food and drink include:

a liquid, emulsified, or pasty food such as a beverage, soy sauce, milk, yogurt, or miso (fermented soybean paste); a semisolid food such as jelly or gummy candy; a solid food such as candy, gum, tofu (soybean curd), or a supplement; and a powdery food.

Examples of the beverage include a fruit juice/fruit beverage, a coffee beverage, an oolong tea beverage, a green tea beverage, a black tea beverage, a barley tea beverage, a vegetable beverage, a carbonated beverage as a soft drink, a fruit extract-containing beverage, a vegetable extract-containing juice, near water (soft drink with minute amounts of flavoring, or the like), a sport beverage, and a diet beverage.

Into the beverage, an additive such as an antioxidant, a flavor, various kinds of esters, an organic acid, an organic acid salt, an inorganic acid, an inorganic acid salt, an inorganic salt, a dye, an emulsifier, a preservative, a seasoning, a sweetener, an acidulant, a fruit juice extract, a vegetable extract, a nectar extract, a pH adjusting agent, or a quality stabilizer can be added alone or in combination thereof.

The food composition provided by the present invention can be used in a similar manner to the method for using a pharmaceutical composition described in "1. Pharmaceutical composition" above, and can also be used in a range not intended to treat or prevent disease. That is, based on citric acid, a pharmaceutically acceptable salt of citric acid, a hydrate of citric acid, a hydrate of the pharmaceutically acceptable salt of citric acid, or a mixture thereof, contained in the food composition according to the present invention, the food composition provided by the present invention can be applied to an application subject of the pharmaceutical composition such that the use amount of citric acid, a pharmaceutically acceptable salt of citric acid, a hydrate of citric acid, a hydrate of the pharmaceutically acceptable salt of citric acid, or a mixture thereof, in the food composition is equal to the amount of citric acid, a pharmaceutically acceptable salt of citric acid, a hydrate of citric acid, a hydrate of the pharmaceutically acceptable salt of citric acid, or a mixture thereof, contained in the pharmaceutical composition. In one embodiment, the "food composition" according to the present invention can be applied to a subject (for example, a human or another mammal) not having a "pathological" or "abnormal" symptom, condition, or disease, that is, a subject (for example, a human or another mammal) in a "healthy" or "normal" condition in order to maintain or promote a "healthy" or "normal" condition. Furthermore, the "food composition" according to the present invention can be applied to a "healthy person who is concerned about protein in urine or kidney health" or a "healthy person who is concerned about a dietary acid load on the kidney" in order to maintain or promote a "healthy" or "normal" condition. In this case, whether the citric acid, the pharmaceutically acceptable salt of citric acid, the hydrate of citric acid, the hydrate of the pharmaceutically acceptable salt of citric acid, or the mixture thereof, is an ingredient of a pharmaceutical composition or an ingredient of a food composition, a pharmacological effect of the citric acid, the pharmaceutically acceptable salt of citric acid, the hydrate of citric acid, the hydrate of the pharmaceutically acceptable salt of citric acid, or the mixture thereof, itself is basically the same. Therefore, the application amount and application method of the food composition can be appropriately adjusted depending on an expected effect based on the citric acid, the pharmaceutically acceptable salt of citric acid, the hydrate of citric acid, the hydrate of the pharmaceutically acceptable salt of citric acid, or the mixture thereof.

The food composition applied to a subject (for example, a human or another mammal) not having a "pathological" or "abnormal" symptom, condition, or disease, that is, a subject (for example, a human or another mammal) in a "healthy" or "normal" condition in order to maintain or promote a "healthy" or "normal" condition may be particularly referred to as a "functionally labeled food".

The term "administration" described in "1. Pharmaceutical composition" above can be applied also to the "food composition" according to the present invention, and furthermore, the term "administration" can be replaced with "ingestion" in the "food composition" according to the present invention. Accordingly, for example, the term "administer", "administered", or the like can be replaced with "allowing ... to ingest", "ingest", "ingested", or the like, while inflecting the word depending on the context.

Therefore, examples of the embodiment of the food composition according to the present invention include the following ones:

(1-1) A food composition containing citric acid, a pharmaceutically acceptable salt of citric acid, a hydrate of citric acid, a hydrate of the pharmaceutically acceptable salt of citric acid, or a mixture thereof, and used for decreasing albumin in spot urine, decreasing albumin in early morning urine, decreasing protein in spot urine, increasing net acid excretion, protecting renal function, or reducing the dietary acid load on the kidney;

(2-1) A method for decreasing albumin in spot urine, decreasing albumin in early morning urine, decreasing protein in spot urine, or protecting renal function, the method including causing a subject in need of decreasing albumin in spot urine, decreasing albumin in early morning urine, decreasing protein in spot urine, or protecting renal function, to ingest a food composition containing an effective amount of citric acid, a pharmaceutically acceptable salt of citric acid, a hydrate of citric acid, a hydrate of the pharmaceutically acceptable salt of citric acid, or a mixture thereof;

(2-2) The method according to (2-1), in which the subject in need of decreasing albumin in spot urine, decreasing albumin in early morning urine, decreasing protein in spot urine, or protecting renal function is a healthy person who is concerned about protein in urine or kidney health;

(2-3) A method for increasing net acid excretion, the method including causing a subject in need of increasing net acid excretion to ingest a food composition containing an effective amount of citric acid, a pharmaceutically acceptable salt of citric acid, a hydrate of citric acid, a hydrate of the pharmaceutically acceptable salt of citric acid, or a mixture thereof;

(2-4) The method according to (2-3), in which the subject in need of increasing net acid excretion is a healthy person who is concerned about a dietary acid load on the kidney;

(3-1) A food composition containing citric acid, a pharmaceutically acceptable salt of citric acid, a hydrate of citric acid, a hydrate of the pharmaceutically acceptable salt of citric acid, or a mixture thereof, for use in decreasing albumin in spot urine, decreasing albumin in early morning urine, decreasing protein in spot urine, increasing net acid excretion, protecting renal function, or reducing the dietary acid load on the kidney;

(4-1) Use of citric acid, a pharmaceutically acceptable salt of citric acid, a hydrate of citric acid, a hydrate of the pharmaceutically acceptable salt of citric acid, or a mixture thereof, for producing a food composition for decreasing albumin in spot urine, decreasing albumin in early morning urine, decreasing protein in spot urine, increasing net acid excretion, protecting renal function, or reducing the dietary acid load on the kidney;

A packaging, a container, or an instruction of the food composition according to the present invention preferably indicates the effect of decreasing albumin in spot urine, decreasing albumin in early morning urine, decreasing protein in spot urine, increasing net acid excretion, protecting renal function, or reducing the dietary acid load on the kidney.

The present invention will be described below in further detail with reference to Examples, but the present invention is not limited thereto.

Examples

In order to examine whether or not the action of decreasing albumin in spot urine, decreasing albumin in early morning urine, decreasing protein in spot urine, or increasing net acid excretion is observed by oral administration of a potassium citrate/sodium citrate hydrate blending formulation or a sodium bicarbonate formulation as an oral alkalizing agent, a human clinical test was performed.

1. Method

93 patients with chronic kidney disease of stage G2 to G3b (eGFR: 30 to 89 ml/min/1.73 $m^2$) were randomly divided into a potassium citrate/sodium citrate hydrate blending formulation administration group (group A: 31 patients), a sodium bicarbonate formulation administration group (group B: 31 patients), and a control group (group C: 31 patients). The patients were allocated to the groups such that the age, the sex, presence or absence of diabetes, and eGFR were balanced. Each of the groups received treatment based on "CKD Clinical Guide-Summary of Treatment" (hereinafter referred to as standard treatment).

No alkalizing agent was administered to the control group. To group A, three tablets each containing 231.5 mg of potassium citrate ($C_6H_5K_3O_7 \cdot H_2O$) and 195.0 mg of sodium citrate hydrate ($C_6H_5Na_3O_7 \cdot 2H_2O$) were orally adminis-

tered per day for 24 weeks, while the daily dose was divided into three portions (morning, daytime, and evening). Note that the pH of early morning urine was controlled over time, and in the case where early morning urine had a pH of less than 6.5, the daily dose could be appropriately increased up to six tablets at discretion of a doctor, while the daily dose was divided into three portions (morning, noon, and evening). To group B, three tablets each containing 500 mg of sodium bicarbonate were orally administered per day for 24 weeks, while the daily dose was divided into three portions (morning, daytime, and evening). Note that the pH of early morning urine was controlled over time, and in the case where early morning urine had a pH of less than 6.5, the daily dose could be appropriately increased up to six tablets at discretion of a doctor, while the daily dose was divided into three portions (morning, noon, and evening).

Early morning urine and blood were collected before start of administration and 6 weeks, 12 weeks, and 24 weeks after start of administration, and each sample was stored at -80°C.

The concentration of albumin in spot urine, the concentration of albumin in early morning urine, and the concentration of protein in spot urine were measured using immunoturbidimetry and colorimetry.

Using the following formula, an anion gap in urine (U-Anion gap), the concentration of titratable acid in urine (U-Titratable acid), and the concentration of ammonia in urine (U-NH4) were determined, and net acid excretion (U-NAE) was calculated.

$$U\text{-}NAE = U\text{-}NH_4 + U\text{-}Titratable\ Acid - U\text{-}HCO_3^-$$

$$U\text{-}NH_4 = (osmotic\ pressure\ -2X\ (Na\ concentration\ in\ urine + K\ concentration\ in\ urine) + urea\ nitrogen\ concentration\ in\ urine/2.8 + glucose\ concentration\ in\ urine/18)/2$$

The unit of each of the Na concentration in urine and the K concentration in urine is [mEq/L], and the unit of each of the urea nitrogen concentration in urine and the glucose concentration in urine is [mg/dL].

$$U\text{-}Titratable\ acid = U\text{-}NH_4 + Anion\ gap$$

$$U\text{-}Anion\ gap = Na\ concentration\ in\ urine + K\ concentration\ in\ urine - Cl\ concentration\ in\ urine$$

The concentration of creatinine in urine was measured by an enzyme method.

Statistical analysis used Mann-Whitney test for comparison between groups and Wilcoxon test for comparison in change over time.

2. Results

From the measurement results, for each patient in group A (potassium citrate/sodium citrate hydrate blending formulation administration group), group B (sodium bicarbonate formulation administration group), and group C (control group), the following values were calculated:

(i) The value obtained by dividing each of the concentrations of albumin in spot urine, albumin in early morning urine, protein in spot urine, net acid excretion in urine, and bicarbonate ions in urine before start of administration by the concentration of creatinine in urine

(ii) The value obtained by dividing each of the concentrations of albumin in spot urine, albumin in early morning urine, protein in spot urine, net acid excretion in urine, and bicarbonate ions in urine 6 weeks, 12 weeks, and 24 weeks after start of administration by the concentration of creatinine in urine

(iii) The ratio between the value obtained by dividing each of the concentrations of albumin in spot urine, albumin in early morning urine, and protein in spot urine 6 weeks, 12 weeks, and 24 weeks after start of administration by the concentration of creatinine in urine and the value obtained by dividing each of the concentrations of albumin in spot urine, albumin in early morning urine, and protein in spot urine before start of administration by the concentration of creatinine in urine (the value obtained by dividing each of the concentrations of albumin in spot urine, albumin in early morning urine, and protein in spot urine 6 weeks, 12 weeks, and 24 weeks after start of administration by the concentration of creatinine in urine/a value obtained by dividing each of the concentrations of albumin in spot urine, albumin in early morning urine, and protein in spot urine before start of administration by the concentration of creatinine in urine)

(iv) The change in amount of the value obtained by dividing each of the concentrations of albumin in spot urine, albumin in early morning urine, protein in spot urine, net acid excretion in urine, and bicarbonate ions in urine 6 weeks, 12 weeks, and 24 weeks after start of administration by the concentration of creatinine in urine from the value before start of administration

Then, for the above (i) to (iv), a mean value and SD in each group were calculated.

The results are presented in the following Tables. Note that group A: potassium citrate/sodium citrate hydrate blending formulation administration group was described as "Citrate", and group B: sodium bicarbonate formulation administration group was described as "Bicarbonate".

Table 1-1-1-1: The value obtained by dividing the concentration of albumin in spot urine by the concentration of creatinine in urine (mg/gCr)

Table 1-1-1-2: The ratio between the value obtained by dividing the concentration of albumin in spot urine 6 weeks, 12 weeks, or 24 weeks after start of administration by the concentration of creatinine in urine and the value obtained by dividing the concentration of albumin in spot urine before start of administration by the concentration of creatinine in urine (%)

Table 1-1-1-3: The change in amount of the value obtained by dividing the concentration of albumin in spot urine by the concentration of creatinine in urine from the value before start of administration (mg/gCr)

Table 1-2-1-1: The value obtained by dividing the concentration of protein in spot urine by the concentration of creatinine in urine (mg/gCr)

Table 1-2-1-2: The ratio between the value obtained by dividing the concentration of protein in spot urine 6 weeks, 12 weeks, or 24 weeks after start of administration by the concentration of creatinine in urine and the value obtained by dividing the concentration of protein in spot urine before start of administration by the concentration of creatinine in urine (%)

Table 1-2-1-3: The change in amount of the value obtained by dividing the concentration of protein in spot urine by the concentration of creatinine in urine from the value before start of administration (mg/gCr)

Table 2-1-1-1: The value obtained by dividing the concentration of albumin in early morning urine by the concentration of creatinine in urine (mg/gCr)

Table 2-1-1-2: The ratio between the value obtained by dividing the concentration of albumin in early morning urine 6 weeks, 12 weeks, or 24 weeks after start of administration by the concentration of creatinine in urine and the value obtained by dividing the concentration of albumin in early morning urine before start of administration by the concentration of creatinine in urine (%)

Table 2-1-1-3: The change in amount of the value obtained by dividing the concentration of albumin in early morning urine by the concentration of creatinine in urine from the value before start of administration (mg/gCr)

Table 1-3: The change in amount of the value obtained by dividing the concentration of net acid excretion in urine 6 weeks, 12 weeks, or 24 weeks after start of administration by the concentration of creatinine in urine from the value before start of administration (mEq/gCr)

Table 1-3-2: The change in amount of the value obtained by dividing the concentration of bicarbonate ions in urine 6 weeks, 12 weeks, or 24 weeks after start of administration by the concentration of creatinine in urine from the value before start of administration (mEq/gCr)

[Table 1-1-1-1]

Spot Urine Albumin (mg/gCr)

| Group | N | 0W | 6W | 12W | 24W | 6-24W (88-92) |
|---|---|---|---|---|---|---|
| Control | 30-31 | 149 ± 363 (18.7) | 200 ± 531 (13.1) | 187 ± 496 (25.3) | 209 ± 466 (15.2) | 199 ± 493 (16.8) |
| Citrate | 29-31 | 195 ± 364 (42.9) | 185 ± 386 (36.4) | 170 ± 360 (37.6) | 162 ± 353 (28.6) | 173 ± 363 (33.9) |

(continued)

Spot Urine Albumin (mg/gCr)

| Group | N | 0W | 6W | 12W | 24W | 6-24W (88-92) |
|---|---|---|---|---|---|---|
| Bicarbonate | 27-31 | 156 ± 296 (30.2) | 207 ± 371[b] (52.8) | 222 ± 423[c] (36.9) | 273 ± 523[d] (78.0) | 235 ± 442[a] (54.2) |

Mean +/- SD ( ): Median
[a]p=0.0533 vs Control (Mann-Whitney)
[b]p=0.0493, [c]p=0.0477 and [d]p=0.0081 vs 0 week (Wilcoxon)
Not Significant between Groups (Kruskal-Wallis & Dunn)
(There is no significant difference between groups of 0 week)

[Table 1-1-1-2]

Spot Urine Albumin (mg/gCr, % relative value vs 0 week)

| Group | N | 6W | 12W | 24W[g] | 6-24W[f] (85-91) |
|---|---|---|---|---|---|
| Control | 30-31 | 137 ± 215[e] (94) | 136 ± 80 (115.5) | 133 ± 122[c] (105) | 135 ± 148[a,h] (105) |
| Citrate | 29-31 | 126 ± 96 (102) | 113 ± 70 (105) | 146 ± 162[d,j] (89) | 128 ± 115[b,i] (98) |
| Bicarbonate | 26-29 | 137 ± 76 (134.5) | 164 ± 115 (124) | 194 ± 127 (154) | 166 ± 111 (141) |

Mean +/- SD ( ): Median
[a]p=0.0093, [b]p=0.0035, [c]p=0.0410, [d]p=0.0327 and [e]p=0.0931 vs Bicarbonate (Mann-Whitney)
[f]p=0.0059, [g]p=0.0448 (Kruskal-Wallis) and [h]p=0.0377, [i]p=0.0079, [j]p=0.0602 vs Bicarbonate (Dunn)

[Table 1-1-1-3]

Spot Urine Albumin (mg/gCr, Δmeasured value vs 0 week)

| Group | N | 6W | 12W | 24W[g] | 6-24W[f,l] (85-91) |
|---|---|---|---|---|---|
| Control | 30-31 | 45.7 ± 188 (-1.1) | 38.7 ± 185 (2.7) | 59.8 ± 141 (0.5) | 48.2 ± 171[b] (0.8) |
| Citrate | 29-31 | - 10.4 ± 175 (0.2) | - 32.2 ± 199[e] (0) | - 16.2 ± 169[a,i,k] (- 2.4) | - 19.5 ± 180[c,h,j,m] (- 0.35) |
| Bicarbonate | 26-29 | 40.2 ± 126 (4.9) | 72.5 ± 243 (7.15) | 121 ± 261 (9.2) | 79.0 ± 221 (8.0) |

Mean +/- SD ( ): Median
[a]p=0.0751 vs Control and [b]p=0.0258, [c]p=0.0005, [d]p=0.0097, [e]p=0.0741 vs Bicarbonate (Mann-Whitney)
[f]p=0.0018, [g]p=0.0226 (Kruskal-Wallis) and [h]p=0.0010, [i]p=0.0185 vs Bicarbonate (Dunn)
[j]p=0.0101 and [k]p=0.0635 vs Control (Student-t)
[l]p=0.0025 (1 way ANOVA) and [m]p=0.0467 vs Control (Tukey)

In group A (Citrate, potassium citrate/sodium citrate hydrate blending formulation administration group), the value of the concentration of albumin in spot urine 6 weeks, 12 weeks, or 24 weeks after administration was lower than those in group B (Bicarbonate, sodium bicarbonate formulation administration group) and group C (Control, control group) (see Table 1-1-1-1). In group A, the concentration of albumin in spot urine 6 to 24 weeks after administration was lower those in groups B and C (see Table 1-1-1-1). The change in amount of the concentration of albumin in spot urine 6 weeks, 12 weeks, or 24 weeks after administration and the change in amount thereof 6 to 24 weeks after administration from the value before start of administration increased in groups B and C, whereas the change in amounts decreased in group A (see Table 1-1-1-3). Therefore, this indicates that administration of the potassium citrate/sodium citrate hydrate blending formulation is useful for protecting renal function as compared with administration of the sodium bicarbonate formulation or the control group.

[Table 1-2-1-1]

Spot Urine Protein (mg/gCr)

| Group | N | 0W | 6W | 12W | 24W | 6-21W[f] (89-90) |
|---|---|---|---|---|---|---|
| Control | 29-31 | 28.0 ± 56.9 (11.5) | 34.5 ± 80.1 (7.9) | 34.0 ± 73.9 (9.95) | 36.7 ± 73.0 (7.0) | 35.1 ± 74.8[a,g] (7.9) |

(continued)

Spot Urine Protein (mg/gCr)

| Group | N | 0W | 6W | 12W | 24W | 6-21W[f] (89-90) |
|---|---|---|---|---|---|---|
| Citrate | 29-31 | 32.2 ± 50.9 (10.8) | 30.6 ± 52.9 (12.1) | 29.6 ± 51.9 (12.0) | 29.0 ± 48.7[c] (10.9) | 29.5 ± 50.7[b] (11.55) |
| Bicarbonate | 27-31 | 25.8 ± 41.7 (12.1) | 35.0 ± 54.6[d] (15.5) | 36.5 ± 60.5 (14.1) | 42.8 = 69.8[e] (17.0) | 38.2 ± 61.7 (14.8) |

Mean +/- SD ( ): Median
[a]p=0.0181, [b]p=0.0707 and [c]p=0.0970 vs Bicarbonate (Mann-Whitney)
[d]p=0.0334 and [e]p=0.0020 vs 0 week (Wilcoxon)
[f]p=0.0419 (Kruskal-Wallis) and [g]p=0.0421 vs Bicarbonate (Dunn)
(There is no significant difference between groups of 0 week)

[Table 1-2-1-2]

Spot Urine Protein (mg/gCr, % relative value vs 0 week)

| Group | N | 6W | 12W | 24W | 6-24W[e] (85-90) |
|---|---|---|---|---|---|
| Control | 28-29 | 103 ± 66[d] (92) | 131 ± 89 (104.5) | 127 ± 76 (109) | 120 ± 78[a,f] (100) |
| Citrate | 29-31 | 122 ± 88 (109) | 128 ± 93 (111.5) | 133 ± 121[c] (89) | 128 ± 100[b,g] (104.5) |
| Bicarbonate | 27-31 | 126 ± 63 (107) | 144 ± 92 (119) | 172 ± 116 (158) | 148 ± 95 (122) |

Mean +/- SD ( ): Median
[a]p=0.0159, [b]p=0.0269, [c]p=0.0564 and [d]p=0.0915 vs Bicarbonate (Mann-Whitney)
[e]p=0.0284 (Kruskal-Wallis) and [f]p=0.0618,[g]p=0.0642 vs Bicarbonate (Dunn)

[Table 1-2-1-3]

Spot Urine Protein (mg/gCr, Δmeasured value vs 0 week)

| Group | N | 6W | 12W | 24W[f] | 6-24W[c,k](85-90) |
|---|---|---|---|---|---|
| Control | 28-29 | 6.5 ± 28.0 (- 0.55) | 6.8 ± 29.4 (0.45) | 10.9 ± 24.1 (0.3) | 8.1 ± 27.0[a] (0) |
| Citrate | 29-31 | - 1.6 ± 26.9 (0.5) | - 3.6 ± 30.8 (0.5) | - 1.8 ± 25.6[c,h,j] (- 1.7) | - 2.3 ± 27.6[b,g,i,l] (0.2) |
| Bicarbonate | 27-31 | 6.9 ± 19.0 (1.2) | 10.6 ± 35.9 (2.7) | 17.0 ± 33.6 (4.7) | 11.7 ± 30.8 (2.7) |

Mean +/- SD ( ): Median
[a]p=0.0329, [b]p=0.0258, [c]p=0.0005 and [d]p=0.0097 vs Bicarbonate (Mann-Whitney)
[e]p=0.0018, [f]p=0.0226 (Kruskal-Wallis) and [g]p=0.0010,[h]p=0.0185 vs Bicarbonate (Dunn)
[i]p=0.0124 and [j]p=0.0565 vs Control (Student-t)
[k]p=0.0033 (1 way ANOVA) and [l]p=0.0429 vs Control (Tukey)

In group A (Citrate, potassium citrate/sodium citrate hydrate blending formulation administration group), the value of the concentration of protein in spot urine 6 weeks, 12 weeks, or 24 weeks after administration was lower than those in group B (Bicarbonate, sodium bicarbonate formulation administration group) and group C (Control, control group) (see Table 1-2-1-1). In group A, the concentration of protein in spot urine 6 to 24 weeks after administration was lower those in groups B and C (see Table 1-2-1-1). The change in amount of the concentration of protein in spot urine 6 weeks, 12 weeks, or 24 weeks after administration and the change in amount thereof 6 to 24 weeks after administration from the value before start of administration increased in groups B and C, whereas the change in amounts decreased in group A (see Table 1-2-1-3). Therefore, this indicates that administration of the potassium citrate/sodium citrate hydrate blending formulation is useful for protecting renal function as compared with administration of the sodium bicarbonate formulation or the control group.

[Table 2-1-1-1]

Early Morning Urine Albumin (mg/gCr)

| Group | N | 0W | 6W | 12W | 24W | 6-24W (88-91) |
|---|---|---|---|---|---|---|
| Control | 30-31 | 119 ± 324 (12.8) | 143 ± 380 (8.0) | 121 ± 293 (11.1) | 153 ± 338 (7.9) | 139 ± 335 (8.7) |
| Citrate | 29-31 | 123 ± 233 (143) | 105 ± 232 (10.1) | 102 ± 194 (16.4) | 91 ± 206[a] (8.5) | 99± 209 (14.4) |
| Bicarbonate | 26-31 | 74 ± 129 (13.8) | 113 ± 186 (22.7) | 126 ± 224 (10.3) | 124 ± 245 (19.5) | 121 ± 219 (20.2) |

Mean +/- SD ( ): Median
Not Significant between Groups (Mann-Whitney)
[a]p=0.0179 vs 0 week (Wilcoxon)
Not Significant between Groups (Kruskal-Wallis & Dunn)
(There is no significant difference between groups of 0 week)

[Table 2-1-1-2]

Early Morning Urine Albumin (mg/gCr, % relative value vs 0 week)

| Group | N | 6W | 12W | 24W[d] | 6-24W[c] (88-91) |
|---|---|---|---|---|---|
| Control | 30-31 | 103 ± 79 (77.5) | 112 ± 75 (93.5) | 122 ± 77 (111) | 113 ± 77 (90) |
| Citrate | 29-31 | 95 ± 44 (97) | 144 ± 248 (82.5) | 98 ± 96[b,f] (75.5) | 112 ± 155[a,e] (83) |
| Bicarbonate | 27-31 | 157 ± 163 (93) | 143 ± 121 (108) | 143 ± 98 (118) | 147 ± 127 (113) |

Mean +/- SD ( ): Median
[a]p=0.0151 and [b]p=0.0233 vs Bicarbonate (Mann-Whitney)
[c]p=0.0487, [d]p=0.0699 (Kruskal-Wallis) and [e]p=0.0421,[f]p=0.0794 vs Bicarbonate (Dunn)

[Table 2-1-1-3]

Early Morning Urine Albumin (mg/gCr, ∆measured value vs 0 week)

| Group | N | 6W | 12W | 24W | 6-24W[e](88-91) |
|---|---|---|---|---|---|
| Control | 30-31 | 19.6 ± 102 (- 0.98) | - 2.0 ± 132 (- 0.68) | 34.1 ± 151 (0.72) | 17.4 ± 130 (- 0.47) |
| Citrate | 29-31 | - 18.1 ± 99 (- 0.07) | - 25.4 ± 91[d] (- 1.07) | - 12.8 ± 59[a,c] (- 2.12) | - 18.8 ± 84[b,f,g] (- 0.98) |
| Bicarbonate | 27-31 | 26.9 ± 123 (- 0.53) | 51.3 ± 148 (0.55) | 50.7 ± 136 (0.97) | 43.6 ± 136 (0.74) |

Mean +/- SD ( ): Median
[a]p=0.0508 vs Control and [b]p=0.0108, [c]p=0.0256, [d]p=0.0951 vs Bicarbonate (Mann-Whitney)
[e]p=0.0353 (Kruskal-Wallis) and [f]p=0.0292 vs Bicarbonate (Dunn)
[g]p=0.0272 vs Control (Student-t)

In group A (Citrate, potassium citrate/sodium citrate hydrate blending formulation administration group), the value of the concentration of albumin in early morning urine 6 weeks, 12 weeks, or 24 weeks after administration was lower than those in group B (Bicarbonate, sodium bicarbonate formulation administration group) and group C (Control, control group) (see Table 2-1-1-1). In group A, the concentration of albumin in early morning urine 6 to 24 weeks after administration was lower those in groups B and C (see Table 2-1-1-1). The change in amount of the concentration of albumin in early morning urine 6 weeks, 12 weeks, or 24 weeks after administration and the change in amount thereof 6 to 24 weeks after administration from the value before start of administration increased in groups B and C, whereas the change in amounts decreased in group A (see Table 2-1-1-3). Therefore, this indicates that administration of the potassium citrate/sodium citrate hydrate blending formulation is useful for protecting renal function as compared with administration of the sodium bicarbonate formulation or the control group.

[Table 1-3]

Net Acid Excretion (-Urine $HCO_3^-$, mEq/gCr, $\Delta$measured value vs 0 week)

| Group | N | 6W | 12W | 24W | 6-24W (88-91) |
|---|---|---|---|---|---|
| Control | 30-31 | - 4.99a± 15.80 (- 5.40) | - 3.10 ± 24.21 (- 2.93) | - 7.83 ± 19.49 (- 8.02) | - 5.33 ± 20.00 (- 5.09) |
| Citrate | 29-31 | 1.85 ± 22.50 (- 1.78) | - 1.42 ± 23.08 (- 0.055) | - 1.73 ± 21.55 (- 2.41) | - 0.40 ± 22.21 (- 0.77) |
| Bicarbonate | 26-31 | -3.58 ± 23.75 (-2.75) | - 2.99 ± 24.63 (- 4.40) | - 5.28 ± 24.00 (- 2.19) | - 3.97 ± 23.90 (- 3.49) |

Mean +/- SD ( ): Median
Not Significant between Groups (Mann-Whitney)
Not Significant between Groups (Kruskal-Wallis & Dunn)

In group A (Citrate, potassium citrate/sodium citrate hydrate blending formulation administration group), the change in amount of net acid excretion 6 weeks, 12 weeks, or 24 weeks after administration and the change in amount thereof 6 to 24 weeks after administration from the value before start of administration were increased as compared with those in group B (Bicarbonate, sodium bicarbonate formulation administration group) and group C (Control, control group), whereas the change in amounts were decreased in group B and group C (see Table 1-3). Therefore, this indicates that administration of the potassium citrate/sodium citrate hydrate blending formulation is useful for reducing a dietary acid load on the kidney as compared with administration of the sodium bicarbonate formulation or the control group.

Industrial Applicability

The pharmaceutical composition, food composition, or the like, provided by the present invention can protect renal function or reduce the dietary acid load on the kidney in mammals.

**Claims**

1. A pharmaceutical composition for protecting renal function, comprising citric acid, a pharmaceutically acceptable salt of citric acid, a hydrate of citric acid, a hydrate of the pharmaceutically acceptable salt of citric acid, or a mixture thereof, wherein the pharmaceutical composition is in the form of a tablet.

2. A pharmaceutical composition for reducing the dietary acid load on a kidney, comprising citric acid, a pharmaceutically acceptable salt of citric acid, a hydrate of citric acid, a hydrate of the pharmaceutically acceptable salt of citric acid, or a mixture thereof.

3. The pharmaceutical composition according to claim 1 or 2, which is administered to an early-stage chronic kidney disease patient.

4. The pharmaceutical composition according to any one of claims 1 to 3, wherein the citric acid, the pharmaceutically acceptable salt of citric acid, the hydrate of citric acid, the hydrate of the pharmaceutically acceptable salt of citric acid, or the mixture thereof, is sodium citrate or a hydrate of sodium citrate, potassium citrate or a hydrate of potassium citrate, or a mixture thereof.

5. The pharmaceutical composition according to any one of claims 1 to 4, comprising anhydrous citric acid.

6. The pharmaceutical composition according to any one of claims 1 to 5, which decreases the concentration of protein in urine.

7. The pharmaceutical composition according to any one of claims 1 to 5, which suppresses an increase in concentration of protein in urine associated with progression of chronic kidney disease.

8. The pharmaceutical composition according to claim 6 or 7, wherein the urine is early morning urine.

9. The pharmaceutical composition according to any one of claims 6 to 8, wherein the protein in urine is albumin in urine.

## INTERNATIONAL SEARCH REPORT

| | International application No. |
|---|---|
| | PCT/JP2021/012668 |

**A. CLASSIFICATION OF SUBJECT MATTER**
Int. Cl. A61K31/194(2006.01)i, A61K9/20(2006.01)i, A61P13/12(2006.01)i
FI: A61K31/194, A61P13/12, A61K9/20

According to International Patent Classification (IPC) or to both national classification and IPC

**B. FIELDS SEARCHED**

Minimum documentation searched (classification system followed by classification symbols)
Int. Cl. A61K31/194, A61K9/20, A61P13/12

Documentation searched other than minimum documentation to the extent that such documents are included in the fields searched
Published examined utility model applications of Japan       1922-1996
Published unexamined utility model applications of Japan     1971-2021
Registered utility model specifications of Japan             1996-2021
Published registered utility model applications of Japan     1994-2021

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X | JP 2018-177752 A (TOHOKU UNIVERSITY) 15 November 2018, claims 12, 13, 27, paragraph [0027], examples | 1-9 |
| A | 金内雅夫．食事療法における指標と指導のパラダイムシフト：食事炎症性と食事性酸負荷．日本臨床生理学会雑誌．2019, vol. 49, no. 2, pp. 59-63, pp. 61, 62, "2. Dietary Acid Load", (Journal of Clinical Physiology), non-official translation (KANAUCHI, Masao. Paradigm Shift for Indicators and Guidance in Diet Therapy: Dietary Inflammation and Dietary Acid Load.) | 1-9 |
| P, X | WO 2020/080499 A1 (TOHOKU UNIVERSITY) 23 April 2020, claims, examples | 1-9 |

☐ Further documents are listed in the continuation of Box C. ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 19.04.2021 | 27.04.2021 |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**INTERNATIONAL SEARCH REPORT**

Information on patent family members

International application No.

PCT/JP2021/012668

| Patent Documents referred to in the Report | Publication Date | Patent Family | Publication Date |
|---|---|---|---|
| JP 2018-177752 A | 15.11.2018 | EP 3616721 A1 paragraphs [0007], [0021], examples TW 201838684 A KR 10-2019-0137147 A CN 110799214 A | |
| WO 2020/080499 A1 | 23.04.2020 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018193648 A

- WO 2018193752 A

**Non-patent literature cited in the description**

- **BRITO-ASHURST, I.D. et al.** Bicarbonate supplementation slows progression of CKD and improves nutritional status. *J. Am. Soc. Nephrol.,* 2009, vol. 20, 2075-2084

- **SOUMA T. et al.** Luminal alkalinization attenuates proteinuria-induced oxidative damage in proximal tubular cells. *J. Am. Soc. Nephrol.,* 2011, vol. 22, 635-648
- *Journal of the Japanese Society of Nephrology,* 2012